# EUROPEAN PATENT APPLICATION

(11) **EP 4 230 252 A1**
(43) Date of publication of application: **23.08.2023**
(21) Application number: 23157233.0
(22) Date of filing: 17.02.2023
(51) Int. Cl.: A61N 1/04, A61N 1/36, A61B 5/11, A61B 5/00

(54) **GALVANIC STIMULATION DEVICE**

(30) Priority: 17.02.2022 EP 22157325
(71) Applicant: Besnard, Stéphane, 14320 May-Sur-Orne (FR); Boissee, Fanny, 14440 Douvres la Delivrande (FR)
(72) Inventor: BESNARD, Stéphane, 14320 May-Sur-Orne (FR)
(74) Representative: Touroude & Associates

(57) **Abstract**

Galvanic vestibular stimulation device comprising:
i. a galvanic stimulator configured to send an electrical stimulation signal to a plurality of electrodes,
ii. a recording module configured to measure an electrophysiological activity, preferably selected from electrical brain activity, eyes motion, muscular activity and head or body position,
iii. a signal processor configured to modify the electrical stimulation signal based on the measured electrophysiological activity.

## Description

The present invention relates to the field of vestibular galvanic stimulation.

Vestibular galvanic stimulation is a long-known technique which consists in sending electric messages to a nerve in the ear that maintains balance. This is done by applying a transcutaneous current to the vestibular area of the brain by positioning electrodes behind the external ear of a subject, in an area corresponding to the vestibular systems.

Although it was first considered very promising, notably for treating mental disorder and as an analgesic treatment, the technology eventually fell out of grace due to a lack of evidence pointing toward a reliable neurophysiological effect, and to unsuitable side-effects.

Besides, inner ear problems such as balance disorder or motion sickness do not have satisfying treatments. Medical treatments include acetylleucine, commercialized under the denomination TANGANIL, which mechanism of action remains unknown, and corticoids. However, these treatments only allow to treat symptoms and do not eradicate the issue.

There is thus a need for a method allowing to treat inner ear disorders.

Surprisingly, the inventors found that vestibular galvanic stimulation used with small currents with specific patterns, tuned to the response of the brain through coupling with brain activity measurements, allowed to effectively act on the inner ear and to fully desensitize people to motion sickness when coupled to virtual reality, or to partially reverse presbyvestibulia, among other beneficial effects.

The present invention relates to a galvanic vestibular stimulation device comprising:
i. a galvanic stimulator configured to send an electrical stimulation signal to a plurality of electrodes,
ii. a recording module configured to measure an electrophysiological activity, preferably selected from electrical brain activity, eyes motion, muscular activity and head or body position,
iii. a signal processor configured to modify the electrical stimulation signal based on the measured electrophysiological activity.

The electrical stimulation sent preferably has an intensity inferior or equal to 10 mA.

Preferably, the electric stimulation signal has a frequency comprised between 0 Hz and 200 Hz, more preferably 1Hz. However high frequency, i.e. more than 10 Hz, multi frequential signals have also proven to be very efficient stimulations.

The signal can include a square stimulus, a wave stimulus, a sinusoidal stimulus, a random stochastic stimulus, and/or any combination thereof.

The signal is sent to a plurality of electrodes. Preferably, the device comprises between 4 and 12 electrodes, more preferably 12 electrodes. That is to say, between one and three pairs of electrodes per ear.

Each pair of electrodes can be positioned on a different orientation. For instance, one pair can be positioned on a horizontal axis whereas another pair is positioned on a vertical axis; a third pair can be positioned diagonally. To ensure a good repartition of the electrodes, they can be positioned regularly on a circle on each side. Having a high number of electrodes positioned on a variety of axis allows for a fine-grained stimulation. Indeed, in a preferred embodiment the electrical stimulation signal delivered to at least one electrode is not the same than the electrical stimulation signal delivered to at least one other electrode.

The signal can be delivered for each ear/each side independently or bilaterally and either synchronously or asynchronously. To control the stimulation, a software can suggest pre-automatic pattern and protocols of delivery or manual programming of stimuli. Sending different currents to each electrode allows very precise stimulation. The signals sent to each electrode can vary in shape, in amplitude or even in frequency.

Even if the signal sent to all electrodes is the same, it will affect the inner ear differently depending on the orientation it takes. Increasing the number of electrodes will thus allow for a more complete stimulation. It is also to be noted that the position of the subject, for instance standing position, sitting position or lying position, can have an effect on the efficiency of the stimulation, therefore providing additional interest to its control by the electrodes.

Any device can be used to measure an electrophysiological activity in response to the stimulation. Eyes motion can be measured by an electrooculogram. Muscular activity can be measured by electromyogram. Head or body position can be measured though 3D accelerometer sensor for balance measure or body position, i.e. stand up or lay down position, which has interest for therapeutic and safety reasons.

The electrical stimulation signal is modified for calibration of efficient stimulus threshold based on the measured electrophysiological activity. Steps b and c can then be repeated so as to form a feedback loop allowing fine tuning of the signal until proper response is measured. Indeed, the threshold of stimulation highly depends on the individual responses as well as on the positioning of the electrodes: even a 1 cm deviation can make a huge difference.

Preferably, the device is designed to be worn on the head or at the neck preferably the device is borne on a headset. Indeed, headsets allows for a relatively precise positioning of the electrodes and avoid the need to position each electrode individually. Alternatively, the device can be borne on a cap or on any suitable headwear.

Preferably, the device according to the invention is configured to send and/or receive an external trigger output so as to synchronize the device with an external system, which can be either a hardware or a software. Conversely, the galvanic vestibular stimulation module may be controlled and triggered by external software.

The device can also be able to send an acoustic stimulation, up to 100 dB, and a vibrating stimulation. Such stimulation can also be updated based on electrophysiological measurements feedback processed by the device according to the invention.

Another object of the present invention is a method for galvanic vestibular stimulation comprising the steps of:
i. Sending an electrical stimulation signal into the vestibule of a patient through a plurality of external cutaneous electrodes,
ii. Measuring an electrophysiological activity of the patient, preferably selected from electrical brain activity, eyes motion, muscular activity and head or body position
iii. Modifying the electrical stimulation signal based on the measured electrophysiological activity.

The stimulation can be done either directly through the hair with contactless cutaneous electrodes or through electrodes stuck on the skin following specific skin cleaning.

The present invention shall be better understood based on the following detailed description of a non-limiting embodiment of the present invention, and on the annexed drawing on which:
- figure 1 is a schematic view of a device according to the present invention,
- figure 2 is a block diagram of the physiological acquisition and vestibular excitation system,
- figure 3 is an exemplary response measured through electrooculogram,
- figure 4 shows different possible electric stimulation signal shapes, and
- figure 5 shows the electrodes location in an alternative embodiment of the invention.

For clarity sake, the relative sizes and proportions of the elements depicted on the drawing have not always been respected, it is understood that the depicted views are merely schematic.

In an exemplary embodiment, a device according to the present invention can be used for research in neurosciences, for exploration and diagnosis of inner ear pathologies, rehabilitation, desensitization to motion sickness, increasing the immersion of virtual reality experience by synchronizing the inner ear with the virtual reality experience and fighting against vestibular sense aging (presbyvestibulia).

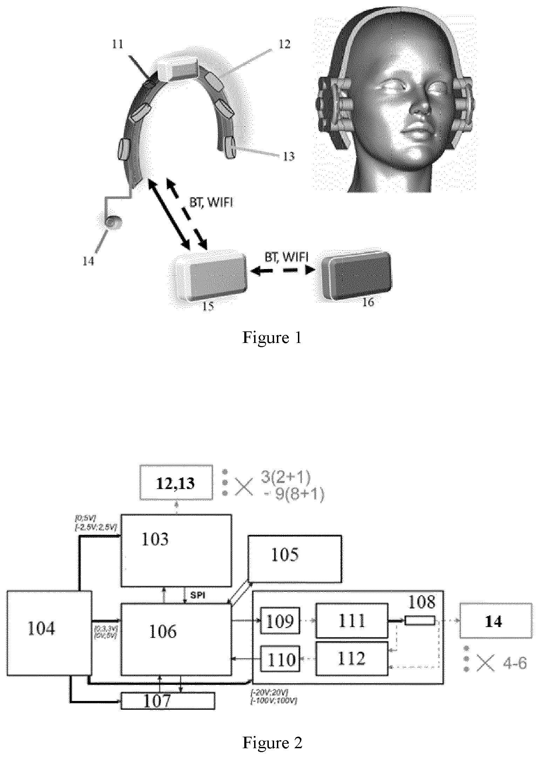

In the exemplary embodiment depicted on Figure 1, six electrodes of stimulation 14 per ear are mounted on a headset. On the same device, one recording electrode 12 is used to gather electrocardiogram (ECG) data (HR) and eight recording electrodes 13 are used for electroencephalogram (EEG) data, electrooculogram (EOG) data and electromyogram data (EMG).

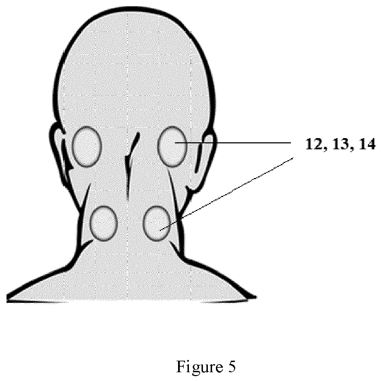

In an alternative embodiment illustrated on figure 5, the electrodes 12, 13, 14 can be located on the mastoid bone, on the neck and/or on the temples. The electrodes can be mounted by an adapted headset or positioned directly on the skin for example through adhesive disposable patches.

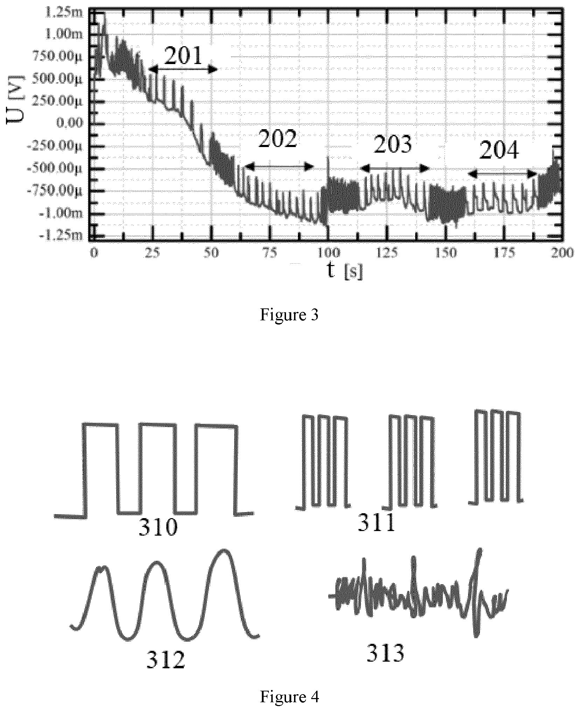

An exemplary EOG acquisition is shown on figure 3. Pattern 201 corresponds to a blinking of both eyes, which corresponds to a reduced basal signal, i.e. eyes motion, when the eyes are closed. Patterns 202 and 203 correspond respectively to movements of left eye and right eye, which also induce a reduced basal signal but only for half of the peaks. Lastly, pattern 204 is the pattern obtained when the subject is asked to move the eyes for a long time.

The headset also has a 3D accelerometer which allows to check whether the user is in vertical position. Indeed, since the device is to be used in the context of balance disorders, it is important to detect falls or loss of balance which may jeopardize the user's safety and require to stop the device's use. It can be coupled to an alarm system so as to alert an external system in case of a detected emergency.

The recording electrodes 12,13 and the electrodes of stimulation 14 are advantageously included in the device. However, in an alternative embodiment, the electrodes can also be separated from the headset, mounted individually and connected to the device. Such configuration allows using classic disposable electrodes such as the one routinely used for EEG or ECG. Such electrodes are to be applied manually on a disinfected skin. Although it requires precise knowledge of the zone to be stimulated, it also allows for a more acute positioning of the electrodes. Alternatively, using contactless electrodes integrated directly into the headset is more user-friendly.

The device can also both hold its own integrated electrodes and be connectable to external electrode if need be.

The device of the exemplary embodiment can communicate with an external controller 15 which holds a program able to operate the device. The communication is wirelessly made, for example through Bluetooth (BT) and/or WIFI and/or any other suitable wireless technology. The program typically gathers all the recorded information. Based on the gathered data, it generates and/or updates an electric stimulation signal which is to be applied by each of the stimulation electrodes 14. The controller also provides any necessary update. The controller can be programmed either directly through a digital screen or through an app in any suitable environment, e.g., the IOS, Android, PC or Mac environment.

The controller can also remotely communicate with a physician or with a system 16 in need of gathering the recorded data and/or able to provide medical indications based on the recorded data.

For instance, a maximum level of stimulation can be set by a medical or paramedical professional. It is also possible to lock the device into a specific mode of stimulation to avoid accidental modification of the stimulation parameters.

Interestingly, all the parameters of the device can be modified by the user in the exemplary embodiment. However, in alternative embodiments, the device can be locked into a given stimulation program; it can allow the user to choose the number of electrodes and the maximum amplitude, or the control can be completely independent from the user and the maximum current delivered by the stimulation can be locked to avoid going 2mA above the maximum for instance.

The parameters of the stimulation are the current delivered, which is comprised between 0.1 and 10mA, the length of the stimulation which can go from 100 ms to several minutes, the frequency, which can be anywhere between stochastic and continuous current but preferably comprised between 0.1 and 400Hz, and the shape of the signal which can be a pulse 311, a ramp, a step 310, a sinusoid 312, completely stochastic 313 or any combination thereof, as shown on figure 4. The stimulation can include pauses during which no current is applied.

Advantageously, the device according to the exemplary embodiment can be triggered through an input/output channel to start the galvanic vestibular stimulation according to a preset protocol based on the desired application: virtual reality, cognitive evaluation, MRI, EEG etc.

The device is advantageously powered by a battery which can be charged either through USB or by through inductive recharging. Indeed, limiting the number of cables is critical in order to provide the subject with as much freedom of movement as possible. In order to increase the device's autonomy, it can also be coupled to an external secondary battery.

In a less preferred embodiment however, the device can be powered through direct USB connection or through a classic plug.

The device comprises six modules which communicate together as shown on figure 2. Dashed arrows correspond to analog signals, bold arrows stand for alimentation and regular arrow for digital signals.

A first block 103 is configured to acquire the physiological signals through the measurement electrodes 12,13. EEG, ECG and EMG signals are converted from analog to digital signal through a converter for ECG, EMG, EEG and bioptentials. The block comprises 1 to 8 channels (24 bits) and a reference potential.

A second block 105 comprises an accelerometer and/or an inertial measurement unit which detects the position of the system and outputs a corresponding digital signal.

A third block 106 serves as a controller. It comprises a microcontroller able to gather the digital outputs from the physiological acquisition block 103 and the accelerometer 105, to send them to an user interface unit, and to generate the shape of the signals for the stimulation while controlling the actual output signals so as to ensure safety of the process.

The stimulation is performed by a fourth block 108 comprising a current source. The digital signal received from the controller block 106 is converted into an analog signal though a digital to analog converter 109 and addressed to an amplifier 111. The current source is powered by a voltage comprised between [-20V ;20V] and [-100V ;100V] to generate currents comprised between 2 and 10 mA, assuming a contact of 10 kOhm. The current is then sent to the stimulation electrodes 14. A follow-up of the current is performed by a dedicated unit 112, which is then transformed into a digital signal through an ADC 110 and addressed back to the controller for adequate monitoring.

Proper powering of each block and battery monitoring - including charge level and usb charge - is done through a dedicated block 104.

A last block 107 is dedicated to the exchange of the data with external systems and handle notably the WIFI and Bluetooth communications.

The device according to the exemplary embodiment is waterproof and made into materials which are inert to ethanol and to most disinfection solutions. Indeed, it is necessary for the device to withhold cleaning and disinfection protocols.

The invention is not limited to the described embodiments. Unless stated otherwise, the expression "comprising one" is to be understood as "comprising at least one" and "or" is to be understood as "and/or'.

## Claims

1. Galvanic vestibular stimulation device comprising:
i. a galvanic stimulator configured to send an electrical stimulation signal to a plurality of electrodes,
ii. a recording module configured to measure an electrophysiological activity, preferably selected from electrical brain activity, eyes motion, muscular activity and head or body position,
iii. a signal processor configured to modify the electrical stimulation signal based on the measured electrophysiological activity.

2. Galvanic vestibular stimulation device according to claim 1 wherein the electric stimulation signal has a frequency comprised between 0 Hz and 200 Hz, preferably 1Hz.

3. Galvanic vestibular stimulation device according to claim 1 or 2 wherein the device is designed to be worn on the head or at the neck preferably wherein the device is borne on a headset.

4. Galvanic vestibular stimulation device according to any which one of the preceding claims wherein the device comprises between 4 and 12 electrodes, preferably 12 electrodes.

5. Galvanic vestibular stimulation device according to any which one of the preceding claims wherein the electrical stimulation signal delivered to at least one electrode is not the same that the electrical stimulation signal delivered to at least one other electrode.

6. Galvanic vestibular stimulation device according to any which one of the preceding claims configured to send and/or receive an external trigger output so as to synchronize the device with an external system.

7. Method for galvanic vestibular stimulation comprising the steps of:
i. Sending an electrical stimulation signal into the vestibule of a patient through a plurality of external cutaneous electrodes,
ii. Measuring an electrophysiological activity of the patient, preferably selected from electrical brain activity, eyes motion, muscular activity and head or body position
iii. Modifying the electrical stimulation signal based on the measured electrophysiological activity.
